(19) Europäisches Patentamt
European Patent Office
Office européen des brevets

(11) Veröffentlichungsnummer: **0 369 394 B1**

(12) **EUROPÄISCHE PATENTSCHRIFT**

(45) Veröffentlichungstag der Patentschrift: **11.08.93**

(51) Int. Cl.$^5$: **A61K 6/10**

(21) Anmeldenummer: **89121056.9**

(22) Anmeldetag: **14.11.89**

# (54) Polyether-Abformmaterial, Verfahren zu seiner Herstellung und seine Verwendung.

(30) Priorität: **14.11.88 DE 3838587**

(43) Veröffentlichungstag der Anmeldung:
**23.05.90 Patentblatt 90/21**

(45) Bekanntmachung des Hinweises auf die Patenterteilung:
**11.08.93 Patentblatt 93/32**

(84) Benannte Vertragsstaaten:
**AT BE CH DE ES FR GB IT LI NL SE**

(56) Entgegenhaltungen:
**EP-A- 0 231 420**
**DE-A- 3 741 575**

(73) Patentinhaber: **THERA Patent GmbH & Co. KG**
**Gesellschaft für industrielle Schutzrechte**
**Am Griesberg 2**
**W-8031 Seefeld 1(DE)**

(72) Erfinder: **Jochum, Peter, Dr.**
**Pointweg 5**
**W-8031 Seefeld 2(DE)**
Erfinder: **Gasser, Oswald, Dr.**
**Höhenstrasse 10**
**W-8031 Seefeld(DE)**
Erfinder: **Zahler, Wolf-Dietrich, Dr.**
**An der Beermahd 5**
**W-8031 Seefeld-Hechendorf(DE)**
Erfinder: **Lechner, Günther, Dr.**
**Hurtenstrasse 8**
**W-8138 Frieding(DE)**
Erfinder: **Guggenberger, Rainer, Dr.**
**Inninger Strasse 13a**
**W-8031 Hechendorf(DE)**
Erfinder: **Ellrich, Klaus, Dr.**
**Auinger Strasse 16**
**W-8031 Wörthsee(DE)**

(74) Vertreter: **Abitz, Walter, Dr.-Ing. et al**
**Abitz, Morf, Gritschneder, Freiherr von Wittgenstein Postfach 86 01 09**
**W-8000 München 86 (DE)**

EP 0 369 394 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Zur Herstellung von Zahnersatz im Dentallaboratorium ist ein Arbeitsmodell, welches die Zahn- und Kieferverhältnisse des Patient so originalgetreu wie möglich wiedergibt, die wichtigste Voraussetzung. Hierzu wird zunächst vom Zahnarzt mittels sogenannter Abformmaterialien eine Negativform im Munde des Patienten hergestellt. Das anfänglich plastisch verformbare Abformmaterial wird dabei mit einem Abdrucklöffel in den Mund des Patienten eingeführt und erstarrt dort zu einem möglichst elastischen Material, das nach dem Herausnehmen die negative Form darstellt. Diese Abformung kann anschliessend mit einem Modellmaterial ausgegossen werden und führt somit zum Arbeitsmodell.

Hochpräzise elastische Abformmaterialien, die sich durch hohe Abformgenauigkeit, hohe Formbeständigkeit und gute Detailwiedergabe auszeichnen, sind beispielsweise Materialien auf Basis Agar-Agar, Polysulfid, Polyether oder die additionsvernetzenden Silikone. Bei den Polyethermaterialien werden aziridinhaltige Substanzen polymerisiert wie sie z.B. in den US-Patentschriften 34 53 242 und 40 93 555 beschrieben sind. Üblicherweise werden neben den aziridinhaltigen Verbindungen auch Füllstoffe, Farbstoffe und weitere Hilfsstoffe eingesetzt. Zur Initiierung der Polymerisationsreaktionen sind die aus der US-PS 41 67 618 bekannten Sulfoniumsalze gut geeignet.

Die Polyethermaterialien sind aufgrund ihres hydrophilen Verhaltens prädestiniert dazu, durch gutes Anfließverhalten auch im feuchten Mundmilieu die Zahnsituation im Mund so exakt wie möglich festzuhalten.

Bei den additionsvernetzenden Silikonabformmaterialien wird die Härtung durch Reaktion eines Polysiloxans mit Vinylendgruppen mit einem Polysiloxan mit SiH-Endgruppen mittels bestimmter Platinkatalysatoren erreicht. Die so erhaltenen Abdrücke zeichnen sich durch sehr gute elastische Eigenschaften und hohe Lagerbeständigkeiten aus. Dagegen ist die Wiedergabegenauigkeit aufgrund des hydrophoben Charakters der Silikone nur als bedingt gut zu bezeichnen.

Zur Verbesserung des hydrophilen Verhaltens von Silikonabdruckmaterialien ist deswegen vorgeschlagen worden, den additionsvernetzenden Silikonabdruckmaterialien polyethoxylierte Oligosiloxane oder Perfluoralkanzusätze hinzuzufügen (z.B. WO 87/03001 oder EP-A 0 231 420). Diese Zusätze bewirken in der Tat eine Verbesserung des Kontaktwinkels eines Wassertropfens auf dem Abformmaterial. Die bessere Benetzbarkeit tritt jedoch mit einer deutlichen Zeitverzögerung ein, so daß sich diese Verbesserung auf das Anfließen im Munde nur ungenügend auswirkt.

In der DE-A 37 41 575 sind härtbare Abformmaterialien beschrieben, die neben ungesättigten Polyethern mit endständigen Alkenylresten auch die Umsetzungsprodukte solcher substituierter Polyether mit Oligosiloxanresten mit mindestens zwei SiH-Gruppen im Molekül sowie Platinkatalysatoren als Hauptbestandteile enthalten. Sie sollen als Zahnabdruckmaterial Verwendung finden und verfügen über gute elastische und sehr hydrophile Eigenschaften. Die beschriebenen siloxanhaltigen SiH-Komponenten sind jedoch wegen ihrer starken Neigung zur Vernetzung schon während der Herstellung schwierig zu isolieren und die Lagerstabilität der diese enthaltenden Abformmaterialien ist aus diesem Grunde nur begrenzt.

Aufgabe der vorliegenden Erfindung ist die Bereitstellung eines elastischen und lagerstabilen additionsvernetzenden Polyether-Abformmaterials.

Gelöst wird diese Aufgabe durch ein Polyether-Abformmaterial, enthaltend

(a) mindestens einen Polyether, welcher mindestens zwei, gegebenenfalls substituierte Vinyl- und/oder Allylendgruppen aufweist,

(b) eine SiH-Komponente,

(c) mindestens einen Platinkatalysator

und gegebenenfalls

(d) übliche Zusatzstoffe,

das dadurch gekennzeichnet ist, daß die SiH-Komponente (b) erhältlich ist durch Umsetzung einer mindestens bifunktionellen Allyl- oder Vinyl-Kohlenwasserstoffverbindung, deren Kohlenwasserstoffrest ohne Berücksichtigung der Allyl-oder Vinylgruppen und gegebenenfalls vorhandener Alkylenethergruppen 6-30 C-Atome aufweist und der mindestens einen aromatisch ungesättigten, heterocyclischen oder cycloaliphatischen Ring enthält, mit mindestens einem Mol pro Vinyl-oder Allylgruppe einer mindestens bifunktionellen SiH-Verbindung der Formeln

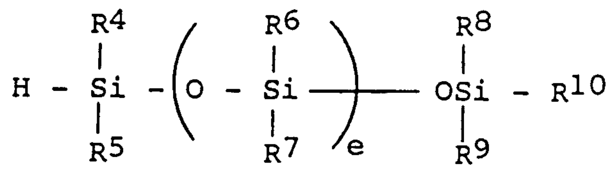

oder

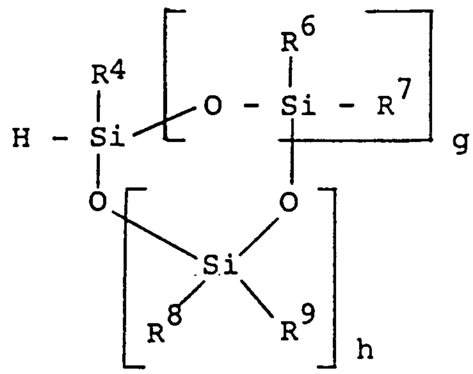

in welchen
e = 0 bis 8,
g = 0 bis 8,
h = 0 bis 4 und
$R^4$ bis $R^{10}$, die gleich oder verschieden sein können, H, Methyl oder Ethyl bedeuten, wobei mindestens einer der Reste $R^4$ bis $R^{10}$ und maximal 5 dieser Reste die Bedeutung von H haben und g und h nicht gleichzeitig O sein können, und wobei mindestens die Komponenten (b) und (c) räumlich voneinander getrennt vorliegen.

Das erfindungsgemäße Abformmaterial hat den Vorteil, daß die enthaltene SiH-Komponente nicht zum Vernetzen neigt und es daher eine zufriedenstellende Lagerstabilität aufweist. Außerdem hat es im Vergleich zu dem aus der DE-A-37 41 575 bekannten Abformmaterial ausgewogenere hydrophile Eigenschaften, indem es einerseits sehr leicht durch Wasser benetzbar ist und damit ein gutes Anfließverhalten zeigt, andererseits aber in Anwesenheit von Wasser nur wenig zum Quellen neigt, d.h. dimensionsstabil bleibt.

Vorzugsweise ist die Allyl- oder Vinyl-Kohlenwasserstoffverbindung eine Allylether-, Vinylether-, Allylester- oder Vinylester-Kohlenwasserstoffverbindung.

Als Komponente (b) werden vorzugsweise SiH-Verbindungen der Formel

$$A \left[ \left( O-B \right)_n - O - C' \right]_m$$

eingesetzt, in welcher

- A einen geradkettigen oder verzweigten 2- bis 6-wertigen Kohlenwasserstoffrest mit 6 bis 30 C-Atomen, enthaltend mindestens einen aromatisch ungesättigten oder cycloaliphatischen Ring,
- B einen geradkettigen oder verzweigten gesättigten Kohlenwasserstoffrest mit 2 bis 6 C-Atomen,

m = 2 bis 6,
n = 0 bis 25, und
C′ die Reste

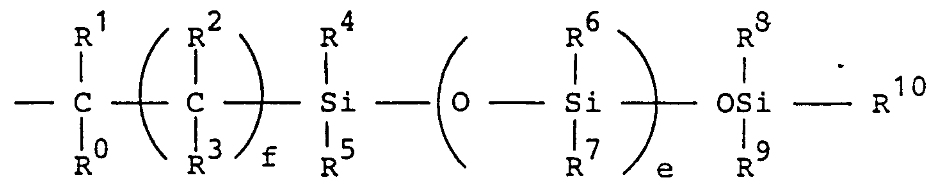

oder

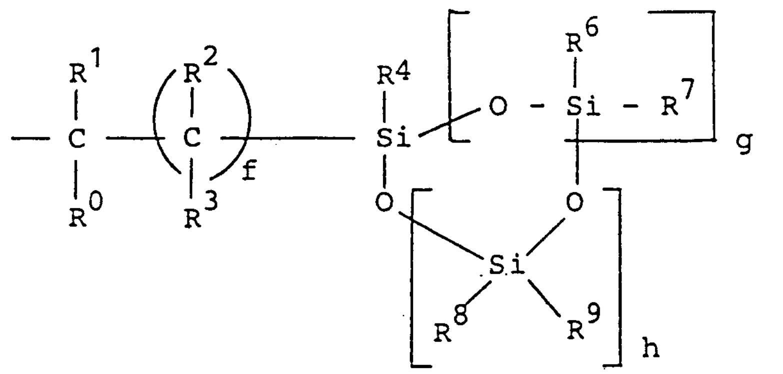

bedeuten, wobei
$R^0$ bis $R^3$, die gleich oder verschieden sein können, H, Methyl oder Ethyl und f 1 oder 2 bedeuten, und e, g, h und $R^4$ bis $R^{10}$ die obige Bedeutung haben.

Das erfindungsgemäße Polyether-Abformmaterial kann so beschaffen sein, daß alle Komponenten (a), (b) und (c) räumlich voneinander getrennt vorliegen. Man kann aber auch die Komponenten (a) und (c) miteinander vermischen und die Komponente (b) hiervon getrennt halten. Bei einer anderen Ausgestaltung kann man einen Teil der Komponente (a) mit der Komponente (c) und getrennt hiervon den restlichen Teil der Komponente (a) mit der Komponente (b) vermischen und die beiden Teilmischungen räumlich voneinander getrennt halten. Kurz vor Gebrauch der Abformmaterialien werden dann die einzelnen Komponenten oder Teilmischungen miteinander vereinigt und gründlich vermischt.

Ein weiterer Gegenstand der Erfindung ist die Verwendung der oben genannten additionsvernetzenden Polyether-Abformmaterialien zur Herstellung von dimensionsstabilen Kieferabdrücken.

Als ungesättigte Polyether (a) können beispielsweise die Di- oder Polyallylether von Polyetherdi- oder -polyolen eingesetzt werden. Als Polyethermittelstück können beispielsweise die Polymeren von Ethylen- und Propylenoxid, Copolymere aus Ethylen- und Propylenoxid sowie Copolymere aus Ethylenoxid und Tetrahydrofuran verwendet werden. Die hieraus erhaltenen Polyetherdiole können dann z.B. mit Allyl- oder auch Vinylchlorid in an sich bekannter Weise zu den ungesättigten Polyethern (a) umgesetzt werden. Die ungesättigten Polyether haben vorzugsweise mittlere Molekulargewichte von 1000 bis 20 000, besonders bevorzugt von 1500 bis 10 000, ganz besonders bevorzugt von 2000 bis 7000. Geeignete ungesättigte Polyether sind in der oben erwähnten DE-A 37 41 575 beschrieben, deren Offenbarung hier insoweit mitumfaßt sein soll.

Komponente (b) des erfindungsgemäßen Abformmaterials ist eine durch Siloxanreste substituierte aromatische oder cycloaliphatische Verbindung. In der obigen Formel für die SiH-Verbindung ist der Rest A vorzugsweise ein zweiwertiger 1,4-Phenylen-, 2,7-Naphthylen-, 4,4'-Isopropylidendiphenylen-, 4,4'-Biphenylylen-, Phthaloyl-, Terephthaloyl- oder Tricyclo-[5.2.1.02,6]-decan-3,8-dimethylenrest.

Rest B ist vorzugsweise ein Ethylen- oder ein Propylenrest, m ist vorzugsweise 2 bis 4, besonders bevorzugt 2, n ist vorzugsweise 0 bis 10, besonders bevorzugt 0 bis 3.

Im Rest C' sind die Reste $R^0$ bis $R^3$ vorzugsweise H oder Methyl, besonders bevorzugt H, und die Reste gleich. f ist vorzugsweise 2, $R^4$ und $R^5$ sind vorzugsweise Methyl. $R^6$ ist vorzugsweise H, $R^7$ und $R^9$ sind vorzugsweise Methyl, $R^8$ und $R^{10}$ sind vorzugsweise H. e ist vorzugsweise 0 bis 5, besonders bevorzugt 1 bis 3. g ist vorzugsweise 1 bis 4 und h ist vorzugsweise 1 bis 2. Besonders bevorzugt sind Reste C' der folgenden Formeln:

$-C_3H_6-Si(Me)_2OSi(Me)_2H$ sowie $-C_2H_4-Si(Me)_2-OSi(Me)_2H$

und folgender Formel:

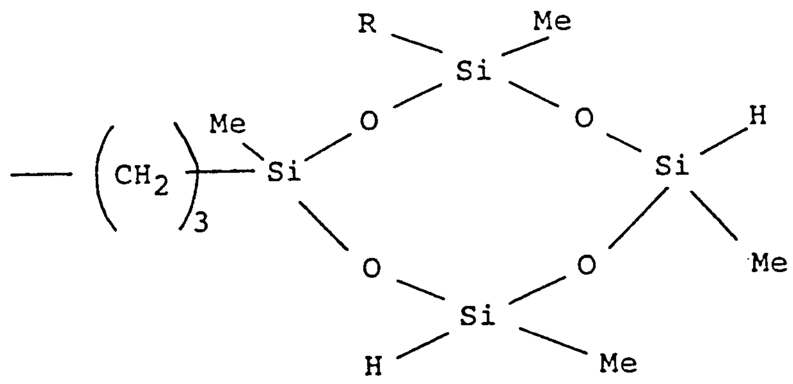

R = H oder Me

Gut geeignet sind beispielsweise auch die Umsetzungsprodukte von Triallyl-triazin-trion mit mindestens der 3-fachen molaren Menge einer mindestens bifunktionellen SiH-Verbindung der oben angegebenen Formeln.

Besonders bevorzugt sind Verbindungen der folgenden Formeln

I.

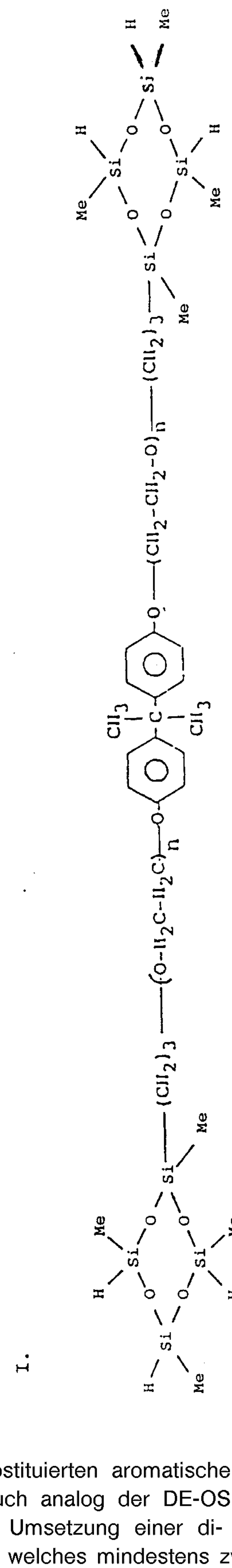

II.

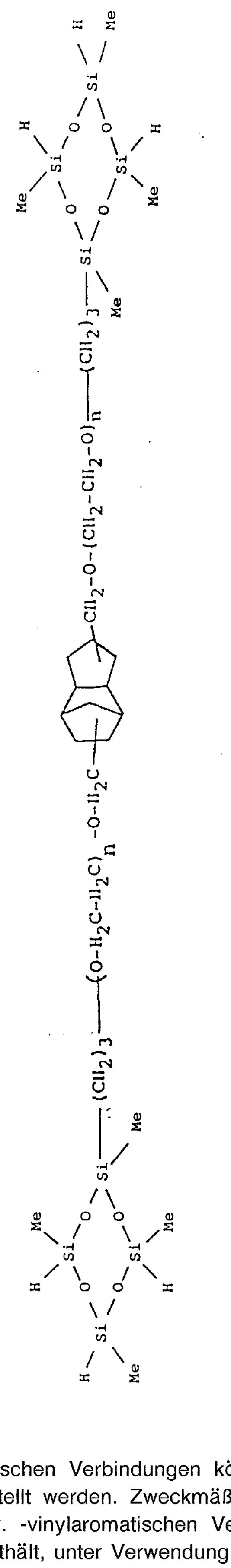

in welchen n 0, 1, 2 oder 3 ist.

Die siloxansubstituierten aromatischen oder cycloaliphatischen Verbindungen können nach üblichen Methoden oder auch analog der DE-OS 37 41 575 hergestellt werden. Zweckmäßigerweise erfolgt die Herstellung durch Umsetzung einer di- oder polyallyl- bzw. -vinylaromatischen Verbindung mit einem Polyorganosiloxan, welches mindestens zwei SiH-Gruppen enthält, unter Verwendung eines Platinkatalysators in einem Mol-Verhältnis von mindestens zwei SiH-Gruppen auf eine Allyl-bzw. Vinylgruppe. Geeignete

Ausgangssubstanzen sind beispielsweise die Diallylether des Bisphenol A, des ethoxylierten Bisphenol A und des Bishydroxymethyl-tricyclo[5.2.1.$0^{2,6}$]-decans sowie auch die Diallylester der Phthal-und Terephthalsäure. Der eingesetzte Katalysator muß zur Herstellung lagerstabiler Pasten entfernt werden, was geeigneterweise durch Adsorption an Kieselgel, Kieselgur oder ähnlichem geschehen kann.

Als Katalysator (c) können alle Katalysatoren, die die Hydrosilylierung einleiten, verwendet werden. Geeignet ist beispielsweise feinverteiltes Platin, Chloroplatinsäure oder Platinkomplexe. Es eignen sich ferner auch alle anderen Verbindungen, die zur Herstellung additionsvernetzender Silikone bekannt sind. Geeignete Platinkomplexe sind Platinolefinkomplexe, so z.B. das Umsetzungsprodukt von Chloroplatinsäure mit einem vinylgruppenhaltigen Polysiloxan.

Das Mol-Verhältnis der Komponente (a) zur Komponente (b) ist zweckmäßigerweise so zu wählen, daß pro 1 Mol ungesättigten Rest 1 bis 10 Mol SiH-Gruppen, vorzugsweise 1,5 bis 3 Mol SiH-Gruppen, vorliegen.

Die eingesetzte Menge an Platinkatalysator (c) beträgt vorzugsweise 0,1 ppm bis 5000 ppm, insbesondere 0,1 bis 1000 ppm, bezogen auf das Gesamtgewicht an Komponente (a) und (b).

Zum Einstellen der Verarbeitungsbedingungen, insbesondere der Fließfähigkeit und Härte der fertigen Abformung, enthält das Abformmaterial gegebenenfalls übliche anorganische und/oder organische Füllstoffe. Geeignete anorganische Füllstoffe sind z.B. pyrogenes Siliciumdioxid, Kieselgur, Kieselgel, Quarzpulver, gemahlene Glasfasern, Titandioxid, Aluminiumoxid, Magnesiumoxid, Calciumcarbonat und Glimmer. Die Kornverteilung der eingesetzten Füllstoffe wird vorzugsweise so gewählt, daß keinerlei Füllstoffe mit Korngrößen > 50 µm enthalten sind; vorzugsweise beträgt die maximale Korngröße 25 µm, besonders bevorzugt 5 µm. Je nach Einsatzzweck beträgt die Menge an Füllstoffen 0 bis 80 Gew.-%, vorzugsweise 5 bis 50 Gew.-%, bezogen auf das Gesamtgewicht des Abformmaterials.

Die Füllstoffe können beschichtet sein. Vorteilhaft sind silanbeschichtete Füllstoffe. Als Silane eignen sich die bekanntlich zur Beschichtung von Füllstoffen eingesetzten Silane. Besonders geeignet sind z.B. Hexamethyl-disilazan und Divinyltetramethyl-disilazan.

Weiterhin kann das erfindungsgemäße Gemisch Zusatzstoffe wie Weichmacher, Pigmente, Antioxidationsmittel, Trennmittel u.ä. enthalten. Als Weichmacher geeignet sind Verbindungen wie Tributylcitrat, Dibenzyltoluol, Polyethylenoxide sowie die Copolymeren aus Ethylen- und Propylenoxid. Die Menge an Weichmacher beträgt vorzugsweise 0 bis 40 Gew.-%, besonders bevorzugt 0 bis 20 Gew.-%, bezogen auf die Gesamtmasse.

Da die Zahl der SiH-Gruppen zur Gewährleistung einer schnellen Abbindung im Verhältnis zur Menge an ungesättigten Resten im härtbaren Abformmaterial relativ groß ist, kann als Nebenprodukt bei der Abbindung Wasserstoffgas frei werden. Um dadurch nicht die Dimensionsstabilität zu beeinflussen, wird vorzugsweise ein Absorber für Wasserstoffgas eingesetzt. Geeignet sind Metallpulver aus Palladium, Platin, Nickel, Magnesium oder Zink; besonders geeignet sind mit solchen Metallen versehene Trägermaterialien, beispielsweise mit Palladium beschichtetes Kieselgel oder mit Palladium bebeschichtetes Calciumcarbonat.

1. Herstellungsbeispiel

Zu 7,92 g Bisallyloxyethylether von Bisphenol-A (20 mMol) werden 7 mg Hexachloroplatinsäure hinzugefügt und der Ansatz 15 Minuten bei Raumtemperatur gerührt, bis sich der größte Teil der Hexachloroplatinsäure gelöst hat. Anschließend werden 9,6 g Tetramethylcyclotetrasiloxan (40 mMol) bei Raumtemperatur langsam hinzugetropft. Dabei erwärmt sich die Mischung innerhalb von 20 Minuten auf eine Temperatur von 55 °C. Es wird so lange weitergerührt, bis sich die Mischung wieder auf eine Temperatur von 30 °C abgekühlt hat und dann wird weitere 2 Stunden gerührt. Anschließend wird über Kieselgur von wenig schwarzem Niederschlag abgesaugt und 10 g einer Verbindung 1 erhalten.

Beispiel 1

Zu 0,27 g von Verbindung 1 werden 1 g des Diallylethers eines Polypropylenglykols mit einem mittleren Molekulargewicht 2000 und 0,2 g einer Platinkatalysatorlösung, bestehend aus 0,1 % Hexachloroplatinsäure in Divinyltetramethyldisiloxan, hinzugefügt und die erhaltene Mischung gerührt. Nach ca. 2 1/2 Minuten setzt die Abbindung ein und ist nach ca. 6 Minuten abgeschlossen. Es wird dabei ein fester Gummi erhalten, der über sehr gute elastische Eigenschaften verfügt und hervorragende Reißfestigkeiten hat.

Das Abformmaterial ist sowohl vor als auch nach der Abbindung gut von Wasser benetzbar, ohne dabei nennenswerte Mengen Wasser aufzunehmen.

2. Herstellungsbeispiel

Wie in Herstellungsbeispiel 1 beschrieben, werden jeweils 20 mMol der in Tabelle 1 angegebenen Diallylverbindungen mit 7 mg Hexachloroplatinsäure und 9,6 g Tetramethylcyclotetrasiloxan zu den in Tabelle 1 angegebenen SiH-Verbindungen umgesetzt. Die Charakterisierung der Produkte erfolgt anhand ihrer FTIR-Spektra. Die SiH-Schwingung des Edukts Tetramethylcyclotetrasiloxan ist bei 2173 $cm^{-1}$ zu sehen. Die Wellenzahlen der erfindungsgemäßen SiH-Verbindungen sind nachfolgend in Tabelle 1 angegeben. Die Vollständigkeit der Umsetzung läßt sich in einfacher Weise durch NMR-Spektroskopie verfolgen. Das breite Multiplet der Allylgruppe bei 5,0 bis 6,3 ppm verschwindet bei vollständiger Umsetzung ganz.

TABELLE 1

| Allylverbindung | Menge (g) | Ausbeute SiH-Verbindung (g) | IR-Spektrum der SiH-Verbindung (SiH-Schwingung) |
|---|---|---|---|
| 4,4′-Bis-(allyloxyethyl-ethoxy)-2,2-diphenylpropan | 13,4 | 18 | 2171 $cm^{-1}$ |
| 4,4′-Bis-(allyloxy)-2,2-diphenylpropan | 6,16 | 6,5 | 2168 $cm^{-1}$ |
| Phthalsäurediallylester | 4,93 | 11 | 2168 $cm^{-1}$ |

Beispiel 2

Die gemäß 2. Herstellungsbeispiel erhaltenen Verbindungen werden, wie in Beispiel 1 beschrieben, mit dem Diallylether eines Polypropylenglykols mit einem mittleren Molekulargewicht 2000 und dem Platinkatalysator, enthaltend 0,1 Gew.-% Hexachloroplatinsäure in Divinyltetramethyldisiloxan, umgesetzt. In jedem Fall setzt die Abbindung nach 2 1/2 Minuten ein und ist nach ca. 6 Minuten abgeschlossen. Die erhaltenen Gummis verfügen über hervorragende Reißfestigkeiten und Elastizitäten.

Das Abformmaterial ist sowohl vor als auch nach der Abbindung gut von Wasser benetzbar, ohne dabei nennenswerte Mengen Wasser aufzunehmen.

Beispiel 3

Aus 2,3 g $H_2PtCl_6$ x 6 $H_2O$ und 11,5 g Tributylcitrat wird durch Rühren bei 100 °C während 5 Minuten eine homogene Lösung erhalten. Diese Lösung läßt man auf 50 °C abkühlen und gibt 13,8 Gewichtsteile Divinyltetramethyldisiloxan hinzu, erhitzt wieder auf 100 °C und rührt für weitere 5 Minuten bei 100 °C. Nach Abkühlen auf Raumtemperatur erhält man eine klare Katalysatorlösung. 0,01 Gewichtsteile dieser Katalysatorlösung werden mit 2 Gewichtsteilen des Diallylethers eines Polypropylenglykols mit einem mittleren Molekulargewicht 2000 sowie 0,02 Gewichtsteilen Palladium auf Calciumcarbonat und 0,9 Gewichtsteilen silanisierter pyrogener Kieselsäure zu einer standfesten Katalysatorpaste verknetet.

0,64 Gewichtsteile des oben beschriebenen Diallylethers werden mit 0,47 Gewichtsteilen des im Herstellungsbeispiel 2 beschriebenen Umsetzungsproduktes aus Tetramethylcyclotetrasiloxan und 4,4-Bis-(allyloxy)-2,2-diphenylpropan sowie 1,9 Gewichtsteilen eines mit Stearinsäure beschichteten Calciumcarbonats zu einer standfesten Basispaste verknetet.

Katalysatorpaste und Basispaste werden in gleichen Gewichtsteilen miteinander vermischt. Nach 2 Minuten 45 Sekunden setzt die Abbindung ein und ist nach ca. 8 Minuten abgeschlossen. Die frisch vermengte Paste verfügt über eine hervorragende Standfestigkeit, läuft nicht aus einem Abformlöffel und läßt sich trotzdem leicht mit einer Elastomerspritze für Dentalabdruckmassen gut um Zähne herum applizieren. Das Material weist nach 30 Minuten eine Shorehärte A von 43 auf, die elastische Verformung beträgt nach 60 Minuten 8 % und die bleibende Deformation nach 60 Minuten 0,5 %. (Die Meßwerte wurden analog ADA 19 für dentale Abformmassen bestimmt.) Das Abformmaterial ist sowohl vor als auch nach der Abbindung gut von Wasser benetzbar, ohne dabei durch Wasseraufnahme zu quellen.

## Patentansprüche

**1.** Polyether-Abformmaterial, enthaltend
(a) mindestens einen Polyether, welcher mindestens zwei, gegebenenfalls substituierte Vinyl- und/oder Allylendgruppen aufweist,

(b) eine SiH-Komponente,
(c) mindestens einen Platinkatalysator,
und gegebenenfalls
(d) übliche Zusatzstoffe,

dadurch gekennzeichnet, daß die SiH-Komponente (b) erhältlich ist durch Umsetzung einer mindestens bifunktionellen Allyl- oder Vinyl-Kohlenwasserstoffverbindung, deren Kohlenwasserstoffrest ohne Berücksichtigung der Allyl-oder Vinylgruppen und gegebenenfalls vorhandener Alkylenethergruppen 6-30 C-Atome aufweist und der mindestens einen aromatisch ungesättigten, heterocyclischen oder cycloaliphatischen Ring enthält, mit mindestens einem Mol pro Vinyl- oder Allylgruppe einer mindestens bifunktionellen SiH-Verbindung der Formeln

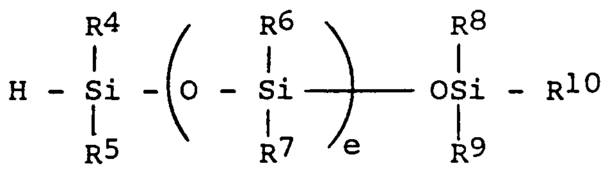

oder

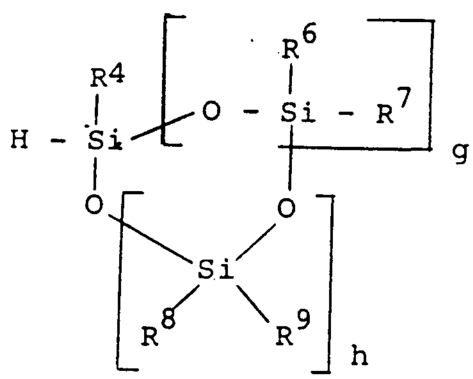

in welchen
e = 0 bis 8,
g = 0 bis 8,
h = 0 bis 4 und
$R^4$ bis $R^{10}$, die gleich oder verschieden sein können, H, Methyl oder Ethyl bedeuten, wobei mindestens einer der Reste $R^4$ bis $R^{10}$ und maximal 5 dieser Reste die Bedeutung von H haben und g und h nicht gleichzeitig O sein können und wobei mindestens die Komponenten (b) und (c) räumlich voneinander getrennt vorliegen.

**2.** Polyether-Abformmaterial nach Anspruch 1, dadurch gekennzeichnet, daß die Allyl- oder Vinyl-Kohlenwasserstoffverbindung eine Allylether-, Vinylether-, Allylester- oder Vinylester-Kohlenwasserstoffverbindung ist.

**3.** Polyether-Abformmaterial nach den Ansprüchen 1 oder 2, dadurch gekennzeichnet, daß die Komponente (b) eine SiH-Verbindung der Formel

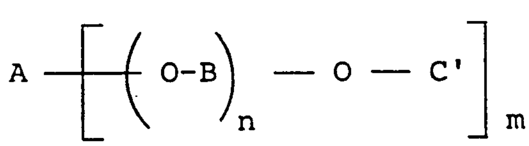

ist, in welcher

A einen geradkettigen oder verzweigten 2- bis 6-wertigen Kohlenwasserstoffrest mit 6 bis 30 C-Atomen, enthaltend mindestens einen aromatisch ungesättigten oder cycloaliphatischen Ring,

B einen geradkettigen oder verzweigten gesättigten Kohlenwasserstoffrest mit 2 bis 6 C-Atomen,

m = 2 bis 6,

n = 0 bis 25, und

C′ die Reste

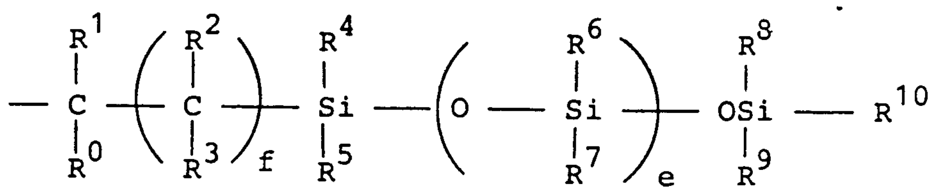

oder

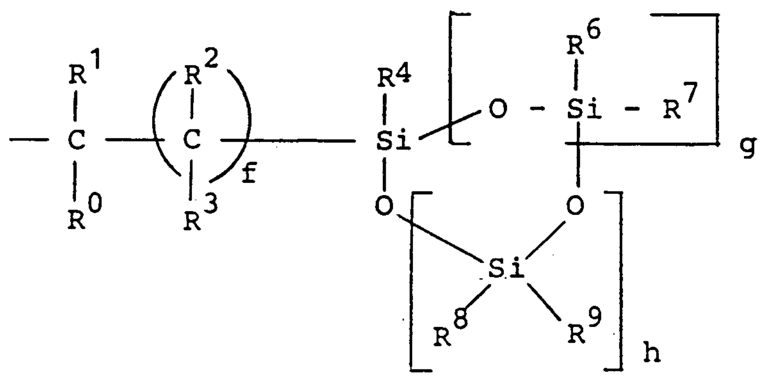

bedeuten, wobei

$R^0$ bis $R^3$, die gleich oder verschieden sein können, H, Methyl oder Ethyl und f 1 oder 2 bedeuten, und e, g, h und $R^4$ bis $R^{10}$ die in Anspruch 1 angegebene Bedeutung besitzen.

**4.** Polyether-Abformmaterial nach den Ansprüchen 1 bis 3, dadurch gekennzeichnet, daß der in Anspruch 1 genannte, ohne Berücksichtigung der Allyl- oder Vinylether- oder -estergruppen und gegebenenfalls vorhandener weiterer Alkylenethergruppen verbleibende Kohlenwasserstoffrest und/oder der in der Formel für die SiH-Verbindung von Anspruch 3 dargestellte Rest A einen 2-wertigen 1,4-Phenylen-, 2,7-Naphthylen-, 4,4′-Isopropylidendiphenylen-, 4,4′-Biphenylylen-, Phthaloyl-, Terephthaloyl- oder Tricyclo-[5.2.1.$0^{2,6}$]-decan-3,8-dimethylenrest bedeuten.

**5.** Polyether-Abformmaterial nach den Ansprüchen 1 bis 4, dadurch gekennzeichnet, daß das Molverhältnis von Komponente (a) zu Komponente (b) so eingestellt wird, daß pro 1 Mol ungesättigten Rest des Polyethers (a) 1 bis 10 Mol, vorzugsweise 1,5 bis 3 Mol, SiH-Gruppen der SiH-Komponente (b) vorliegen.

**6.** Polyether-Abformmaterial nach den Ansprüchen 1 bis 5, dadurch gekennzeichnet, daß der Platinkatalysator (c) in Form von feinverteiltem Platin, Chloroplatinsäure oder Platinkomplexen vorliegt.

**7.** Polyether-Abformmaterial nach den Ansprüchen 1 bis 6, dadurch gekennzeichnet, daß die Menge an Platinkatalysator (c) 0,1 ppm bis 5000 ppm, vorzugsweise 0,1 bis 1000 ppm, bezogen auf das Gesamtgewicht der Komponenten (a) und (b), beträgt.

**8.** Verfahren zur Herstellung des Polyether-Abformmaterials nach den Ansprüchen 1 bis 6, dadurch gekennzeichnet, daß man die Komponenten (a) und (c) miteinander vermischt und die Komponente (b) von der Mischung räumlich getrennt hält.

**9.** Verfahren zur Herstellung des Polyether-Abformmaterials nach den Ansprüchen 1 bis 6, dadurch gekennzeichnet, daß man einen Teil der Komponente (a) mit der Komponente (c) und getrennt hiervon den restlichen Teil der Komponente (a) mit der Komponente (b) vermischt und beide Teilmischungen räumlich voneinander getrennt hält.

**10.** Verwendung des Polyether-Abformmaterials nach den Ansprüchen 1 bis 7 zur Herstellung von dimensionsstabilen Kieferabdrücken.

**Claims**

**1.** Polyether-moulding material, containing

(a) at least one polyether, which has at least two, optionally substituted vinyl- and/or allyl end groups,

(b) a SiH component

(c) at least one platinum catalyst,

and optionally

(d) usual additives,

characterized in that the SiH component (b) can be obtained by the reaction of an at least bifunctional allyl- or vinyl-hydrocarbon compound, the hydrocarbon radical of which, without taking account of the allyl or vinyl groups and the optionally present alkylene ether groups, has 6 - 30 carbon atoms and contains at least one aromatically unsaturated, heterocyclic, or cycloaliphatic ring, with at least one mole per vinyl or allyl group of an at least bifunctional SiH-compound of the formulae

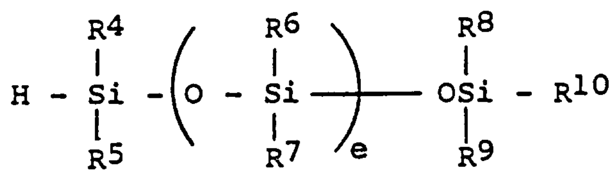

or

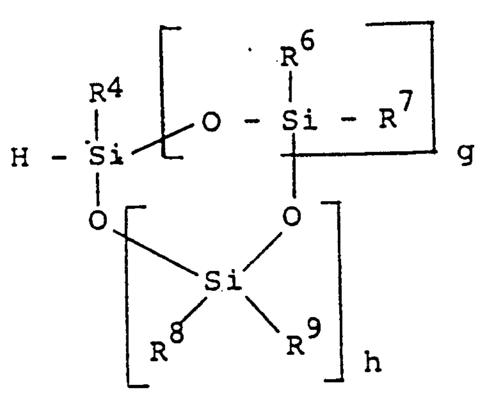

in which

e = 0 to 8

g = 0 to 8

h = 0 to 4 and

$R^4$ to $R^{10}$, which can be the same or different, mean H, methyl or ethyl, while at least one and at most 5 of the radicals $R^4$ to $R^{10}$ mean H and g and h cannot simultaneously be 0, and wherein at least the components (b) and (c) are spatially separated from each other.

**2.** Polyether-moulding material according to claim 1, characterized in that the allyl- or vinyl-hydrocarbon compound is an allylether-, vinylether-, allylester-, or vinylester-hydrocarbon compound.

3. Polyether-moulding material according to claim 1 or 2, characterized in that the component (b) is a SiH-compound of the formula

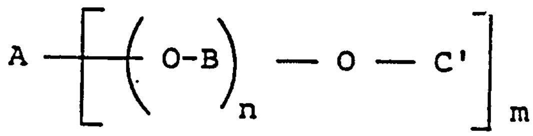

in which

A is a straight-chain or branched 2- to 6-valent hydrocarbon radical with 6 to 30 C-atoms, containing at least one aromatically unsaturated or cycloaliphatic ring,

B is a straight-chain or branched saturated hydrocarbon radical with 2 to 6 carbon atoms,

m = 2 to 6,

n = 0 to 25, and

C' means the radicals

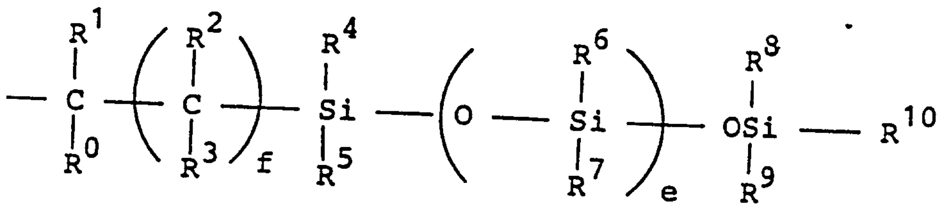

or

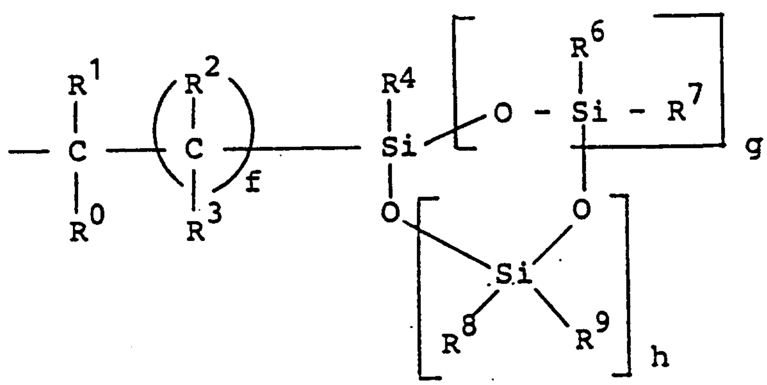

in which

$R^0$ to $R^3$, which can be the same or different, mean H, methyl or ethyl and f means 1 or 2, and e, g, h and $R^4$ to $R^{10}$ have the meaning given in claim 1.

4. Polyether-moulding material according to claims 1 to 3, characterized in that the remaining hydrocarbon radical named in claim 1, without taking account of the allyl- or vinylether- or -ester groups and the further alkylene ether groups optionally present, and/or the radical A shown in the formula for the SiH compound in claim 3 means a 2-valent 1,4-phenylene, 2,7-naphtylene, 4,4'-isopropylidenediphenylene, 4,4'-biphenylylene, phthaloyl, terephthaloyl or tricyclo-[5.2.1.$0^{2,6}$]-decan-3,8-dimethylene radical.

**5.** Polyether-moulding material according to claims 1 to 4, characterized in that the mole ratio of component (a) to component (b) is adjusted in such a way that per 1 mole of unsaturated radical of the polyether (a) there is 1 to 10 moles, preferably 1.5 to 3 moles of the SiH groups of the SiH component (b).

**6.** Polyether-moulding material according to claims 1 to 5, characterized in that the platinum catalyst (c) is present in the form of finely dispersed platinum, chloroplatinic acid or platinum complexes.

**7.** Polyether-moulding material according to claims 1 to 6, characterized in that the quantity of platinum catalyst (c) amounts to 0.1 ppm to 5000 ppm, preferably 0.1 ppm to 1000 ppm based on the total weight of the components (a) and (b).

**8.** Process for the preparation of polyether-moulding material according claims 1 to 6, characterized in that the component (a) and the component (c) are mixed together and the component (b) is spatially separated from the mixture.

**9.** Process for the preparation of polyether-moulding material according to claims 1 to 6, characterized in that a part of component (a) is mixed with the component (c) and separate therefrom the remaining part of component (a) is mixed with the component (b) and both part-mixtures are kept spatially separated from each other.

**10.** Use of polyether-moulding material according to claims 1 to 7 for the production of dimensionally stable dental impressions.

## Revendications

**1.** Matériau d'empreinte à base de polyéther, contenant :

(a) au moins un polyéther qui comporte au moins deux groupes terminaux vinyle et/ou allyle, éventuellement substitués,
(b) un composant SiH,
(c) au moins un catalyseur de platine,
et éventuellement
(d) des additifs usuels,

qui est caractérisé en ce qu'on obtient le composant SiH (b) grâce à la réaction d'un composé hycarboné allyle ou vinyle au moins bifonctionnel, dont le reste hydrocarboné comporte de 6 à 30 atomes de carbone, indépendamment des groupes allyle ou vinyle et de groupes éther d'alkylène éventuellement présents, et qui contient au moins un composé aromatique insaturé, hétérocyclique ou cycloaliphatique, avec, par groupe vinyle ou allyle, au moins une mole d'un composé SiH au moins bifonctionnel, répondant à l'une des formules :

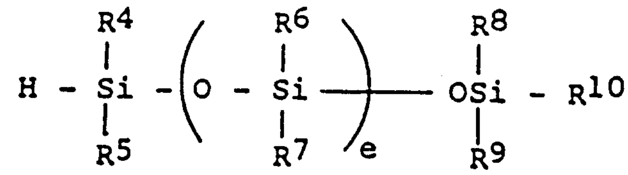

et

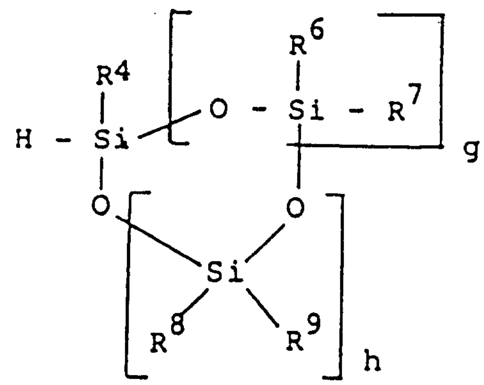

dans lesquelles
e = 0 à 8,
g = 0 à 8,
h = 0 à 4, et
$R^4$ à $R^{10}$, qui peuvent être identiques ou différents, signifient H, méthyle ou éthyle, au moins un des restes $R^4$ à $R^{10}$ et 5 de ces restes au plus ayant la signification de H ; g et h ne pouvant pas valoir 0 en même temps, et au moins les composants (b) et (c) étant spatialement séparés l'un de l'autre.

**2.** Matériau d'empreinte à base de polyéther selon la revendication 1, caractérisé en ce que le composé hydrocarboné allyle ou vinyle est un composé hydrocarboné à base d'éther allylique, d'éther vinylique, d'ester allylique ou d'ester vinylique.

**3.** Matériau d'empreinte à base de polyéther selon les revendications 1 et 2, caractérisé en ce que le composant (b) est un composé SiH répondant à la formule :

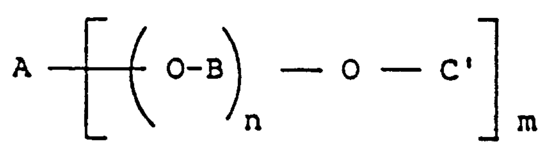

dans laquelle
- A signifie un reste hydrocarboné bifonctionnel à hexafonctionnel, à chaîne droite ou ramifiée, comportant de 6 à 30 atomes de carbone et contenant au moins un cycle aromatique insaturé ou un composé cycloaliphatique,
- B signifie un reste hydrocarboné saturé, à chaîne droite ou ramifiée, contenant de 2 à 6 atomes de carbone,

m = 2 à 6,
n = 0 à 25, et
C' signifie le reste

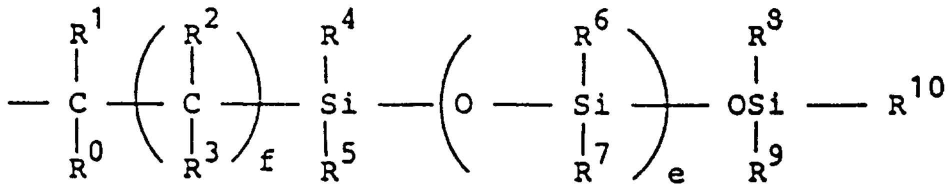

ou

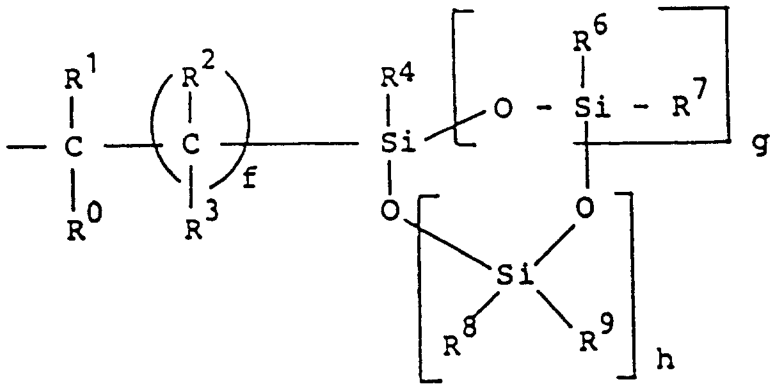

dans lesquels :

$R^0$ à $R^3$, qui peuvent être identiques ou différents, signifient H, méthyle ou éthyle ; f vaut 1 ou 2 ; et e, g, h et $R^4$ à $R^{10}$ ont la signification indiquée dans la revendication 1.

4. Matériau d'empreinte à base de polyéther selon les revendications 1 à 3, caractérisé en ce que le reste hydrocarboné restant, cité dans la revendication 1, sans prendre en considération les groupes éthers ou esters d'allyle ou de vinyle et des groupes éthers d'alkylène supplémentaires éventuellement présents, et/ou le reste A, représenté dans la formule pour le composé SiH de la revendication 3, signifient un reste bifonctionnel 1,4-phénylène, 2,7-naphtylène, 4,4'-isopropylidènediphénylène, 4,4'-biphénylylène, phtaloyle, téréphtaloyle ou tricyclo-[5.2.1. $0^{2,6}$]-décane-3,8-diméthylène.

5. Matériau d'empreinte à base de polyéther selon les revendications 1 à 4, caractérisé en ce que le rapport molaire composant (a) sur composant (b) est réglé de manière telle qu'à 1 mole de reste insaturé du polyéther (a) correspond(ent) 1 à 10 moles, de préférence 1,5 à 3 moles, de groupes SiH du composant SiH (b).

6. Matériau d'empreinte à base de polyéther selon les revendications 1 à 5, caractérisé en ce que le catalyseur de platine (c) se présente sous forme de platine finement divisé, d'acide chloroplatinique ou de complexes de platine.

7. Matériau d'empreinte à base de polyéther selon les revendications 1 à 6, caractérisé en ce que la quantité de catalyseur de platine (c) est de 0,1 ppm à 5 000 ppm, de préférence de 0,1 à 1 000 ppm, par rapport au poids total des composants (a) et (b).

8. Procédé de préparation du matériau d'empreinte à base de polyéther selon les revendications 1 à 6, caractérisé en ce qu'on mélange ensemble les composants (a) et (c) et on garde le composant (b) à l'écart de ce mélange.

9. Procédé de préparation du matériau d'empreinte à base de polyéther selon les revendications 1 à 6, caractérisé en ce qu'on mélange une partie du composant (a) avec le composant (c) et, séparément, on mélange la partie restante du composant (a) avec le composant (b) et on garde les deux mélanges partiels spatialement séparés l'un de l'autre.

10. Utilisation du matériau d'empreinte à base de polyéther selon les revendications 1 à 7 pour la préparation d'empreintes de mâchoires, de dimensions stables.